# EUROPEAN PATENT APPLICATION

(11) **EP 4 119 061 A1**
(43) Date of publication of application: **18.01.2023**
(21) Application number: 21779253.0
(22) Date of filing: 19.03.2021
(51) Int. Cl.: A61B 8/12, A61B 1/313

(54) **IMAGE PROCESSING DEVICE, IMAGE PROCESSING SYSTEM, IMAGE DISPLAY METHOD, AND IMAGE PROCESSING PROGRAM**

(30) Priority: 30.03.2020 JP 2020061493
(71) Applicant: TERUMO Kabushiki Kaisha, Tokyo 151-0072 (JP)
(72) Inventor: SAKAMOTO Yasukazu, Ashigarakami-gun, Kanagawa 259-0151 (JP); SHIMIZU Katsuhiko, Ashigarakami-gun, Kanagawa 259-0151 (JP); ISHIHARA Hiroyuki, Ashigarakami-gun, Kanagawa 259-0151 (JP); HENN Thomas, Sakai-shi, Osaka 599-8231 (JP); JACQUET Clément, Sakai-shi, Osaka 599-8231 (JP); TCHEN Stephen, Sakai-shi, Osaka 599-8231 (JP); SAGA Ryosuke, Sakai-shi, Osaka 599-8231 (JP)
(74) Representative: Casalonga
(86) International application number: PCT/JP2021/011533
(87) International publication number: WO 2021/200294

(57) **Abstract**

An image processing device is an image processing device configured to cause a display to display, as a three-dimensional image, three-dimensional data representing a biological tissue having a longitudinal lumen. The image processing device includes: a control unit configured to calculate centroid positions of a plurality of cross sections in a lateral direction of the lumen of the biological tissue by using the three-dimensional data, set a pair of planes intersecting at a single line passing through the calculated centroid positions as cutting planes, and form, in the three-dimensional data, an opening exposing the lumen of the biological tissue from a region interposed between the cutting planes in the three-dimensional image.

## Description

### Technical Field

The present disclosure relates to an image processing device, an image processing system, an image display method, and an image processing program.

### Background Art

PTL 1 to PTL 3 disclose a technique of generating a three-dimensional image of a cardiac cavity or a blood vessel by using a US image system. The term "US" is an abbreviation for ultrasound.

### Citation list

### Patent Literature

PTL 1: U.S. Patent Application Publication No. 2010/0215238
PTL 2: US Patent No. 6385332
PTL 3: US Patent No. 6251072

### Summary of Invention

### Technical Problem

Treatment using IVUS is widely executed for a cardiac cavity, a cardiac blood vessel, a lower limb artery region, and the like. The term "IVUS" is an abbreviation for intravascular ultrasound. IVUS is a device or a method for providing a two-dimensional image of a plane perpendicular to a long axis of a catheter.

At present, an operator needs to execute treatment while reconstructing a three-dimensional structure by stacking the two-dimensional images of IVUS in his/her head, which is a barrier particularly to young doctors or inexperienced doctors. In order to remove such a barrier, it is conceivable to automatically generate a three-dimensional image expressing a structure of a biological tissue such as the cardiac cavity or the blood vessel from the two-dimensional images of IVUS and display the generated three-dimensional image toward the operator.

However, if the operator can see only an outer wall of the biological tissue in the three-dimensional image, the operator cannot execute treatment inside the biological tissue.

An object of the present disclosure is to enable a user to see an inside of a biological tissue with a three-dimensional image.

### Solution to Problem

An image processing device according to an aspect of the present disclosure is an image processing device configured to cause a display to display, as a three-dimensional image, three-dimensional data representing a biological tissue having a longitudinal lumen. The image processing device includes: a control unit configured to calculate centroid positions of a plurality of cross sections in a lateral direction of the lumen of the biological tissue by using the three-dimensional data, set a pair of planes intersecting at a single line passing through the calculated centroid positions as cutting planes, and form, in the three-dimensional data, an opening exposing the lumen of the biological tissue from a region interposed between the cutting planes in the three-dimensional image.

In one embodiment, when at least a part of the lumen of the biological tissue is bent in a longitudinal direction, the control unit forms, in the three-dimensional data, an opening exposing the bent portion of the lumen in the three-dimensional image over the entire longitudinal direction as the opening.

In one embodiment, the control unit is configured to set the cutting planes after smoothing calculation results of the centroid positions.

In one embodiment, the control unit is configured to divide the calculation results of the centroid positions according to positions of the plurality of cross sections in a longitudinal direction of the lumen of the biological tissue, and smooth each divided calculation result.

In one embodiment, the control unit is configured to adjust a degree of the smoothing executed for the calculation results of the centroid positions according to positions of the plurality of cross sections in a longitudinal direction of the lumen of the biological tissue.

In one embodiment, the image processing device further includes: an input unit configured to receive an operation of a user. The control unit is configured to receive, via the input unit, an operation of setting an angle between the cutting planes.

In one embodiment, the image processing device further includes: an input unit configured to receive an operation of a user. The control unit is configured to receive, via the input unit, an operation of setting an angle for displaying the three-dimensional image, and adjust positions of the cutting planes according to the set angle.

In one embodiment, the biological tissue includes a blood vessel.

An image processing system according to an aspect of the present disclosure includes: a sensor configured to acquire tomographic data of the biological tissue while moving in the lumen of the biological tissue; and the image processing device configured to generate the three-dimensional data based on the tomographic data acquired by the sensor.

In one embodiment, the image processing system further includes: the display.

An image display method according to an aspect of the present disclosure is an image display method for causing a display to display, as a three-dimensional image, three-dimensional data representing a biological tissue having a longitudinal lumen. The image display method includes: a computer calculating centroid positions of a plurality of cross sections in a lateral direction of the lumen of the biological tissue by using the three-dimensional data; the computer setting a pair of planes intersecting at a single line passing through the calculated centroid positions as cutting planes; and the computer forming, in the three-dimensional data, an opening exposing the lumen of the biological tissue from a region interposed between the cutting planes in the three-dimensional image.

An image processing program according to an aspect of the present disclosure causes a computer to execute: processing of causing a display to display, as a three-dimensional image, three-dimensional data representing a biological tissue having a longitudinal lumen; processing of calculating centroid positions of a plurality of cross sections in a lateral direction of the lumen of the biological tissue by using the three-dimensional data; processing of setting a pair of planes intersecting at a single line passing through the calculated centroid positions as cutting planes; and processing of forming, in the three-dimensional data, an opening exposing the lumen of the biological tissue from a region interposed between the cutting planes in the three-dimensional image.

### Advantageous Effect

According to the present disclosure, the user can see an inside of a biological tissue with a three-dimensional image.

### Brief Description of Drawings

[FIG. 1] FIG. 1 is a perspective view of an image processing system according to an aspect of the present disclosure.
[FIG. 2] FIG. 2 is a perspective view of a probe and a drive unit of the image processing system according to the aspect of the present disclosure.
[FIG. 3] FIG. 3 is a block diagram illustrating a configuration of an image processing device according to the aspect of the present disclosure.
[FIG. 4] FIG. 4 is a diagram illustrating a pair of cutting planes set in the aspect of the present disclosure.
[FIG. 5] FIG. 5 is a diagram illustrating one cutting plane set in a comparative example.
[FIG. 6] FIG. 6 is a flowchart illustrating an operation of the image processing system according to the aspect of the present disclosure.
[FIG. 7] FIG. 7 is a flowchart illustrating an operation of the image processing system according to the aspect of the present disclosure.
[FIG. 8] FIG. 8 is a diagram illustrating a result of binarizing a cross-sectional image of a biological tissue in the aspect of the present disclosure.
[FIG. 9] FIG. 9 is a diagram illustrating a result of extracting a point cloud on an inner surface of the biological tissue in the aspect of the present disclosure.
[FIG. 10] FIG. 10 is a diagram illustrating a result of calculating a centroid position of a cross section of the biological tissue in the aspect of the present disclosure.
[FIG. 11] FIG. 11 is a diagram illustrating results of calculating the centroid positions of a plurality of the cross sections of the biological tissue in the aspect of the present disclosure.
[FIG. 12] FIG. 12 is a diagram illustrating a result of smoothing the results of FIG. 11.

### Description of Embodiments

Hereinafter, an embodiment as a specific example according to an aspect of the present disclosure will be described with reference to the drawings.

In the drawings, the same or corresponding parts are denoted by the same reference numerals. In the description of the present embodiment, the description of the same or corresponding parts will be omitted or simplified as appropriate.

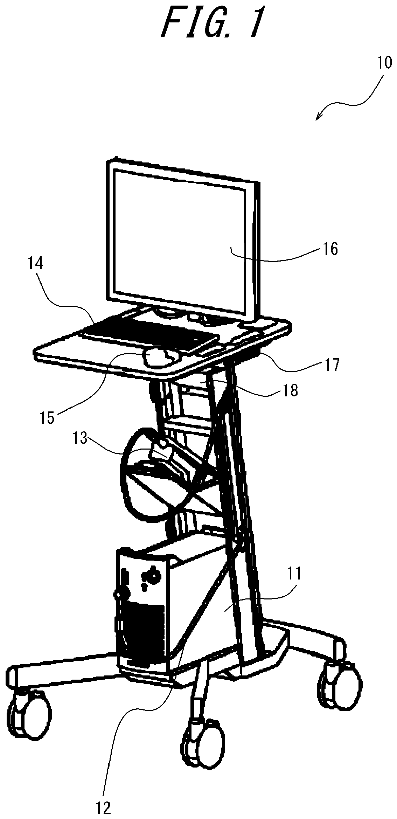

An outline of the present embodiment will be described with reference to FIGS. 1, 3, and 4.

An image processing device 11 according to the present embodiment is a computer that causes a display 16 to display, as a three-dimensional image 53, three-dimensional data 52 representing a biological tissue 60 having a longitudinal lumen. The image processing device 11 calculates centroid positions of a plurality of cross sections in a lateral direction of the lumen of the biological tissue 60 by using the three-dimensional data 52. The image processing device 11 sets a pair of planes intersecting at a single line passing through the calculated centroid positions as cutting planes. The image processing device 11 forms, in the three-dimensional data 52, an opening exposing the lumen of the biological tissue 60 from a region interposed between the cutting planes in the three-dimensional image 53. Note that the biological tissue 60 having a longitudinal lumen as used herein is not limited to an anatomically single organ or a part thereof, but also includes a tissue having a longitudinal lumen across a plurality of organs. An example of such a tissue is specifically a part of a vascular tissue extending from an upper portion of an inferior vena cava to a lower portion of a superior vena cava through a right atrium.

According to the present embodiment, a user can see an inside of the biological tissue 60 with the three-dimensional image 53. For example, when the user is an operator, it is easy to execute treatment for the inside of the biological tissue 60.

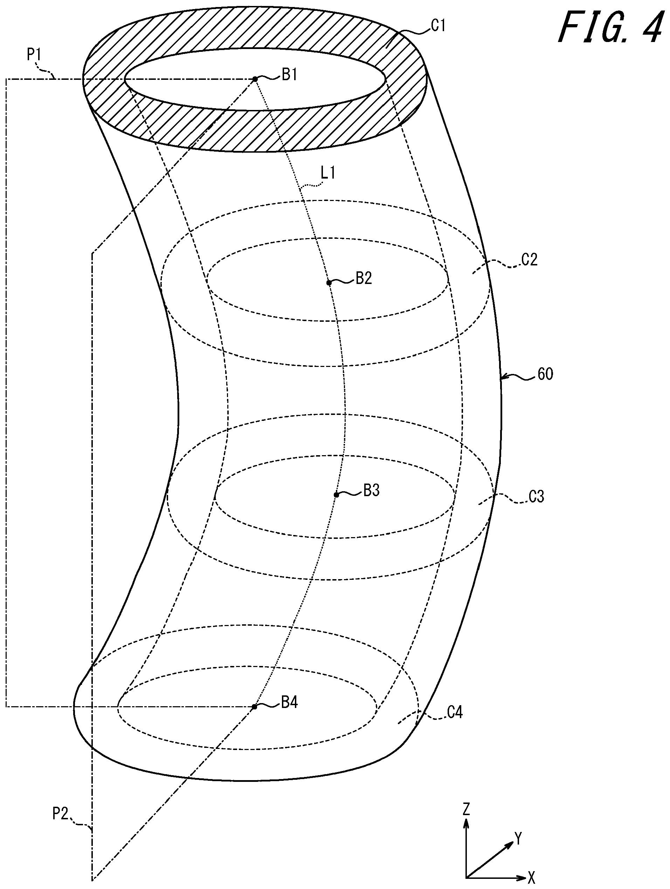

The biological tissue 60 is, for example, an organ such as a blood vessel or a heart. In the example of FIG. 4, the biological tissue 60 is a blood vessel.

In FIG. 4, an X-direction and a Y-direction orthogonal to the X-direction correspond to the lateral direction of the lumen of the biological tissue 60. A Z-direction orthogonal to the X-direction and the Y-direction corresponds to a longitudinal direction of the lumen of the biological tissue 60.

In the example of FIG. 4, the image processing device 11 calculates positions of centroids B1, B2, B3, and B4 of cross sections C1, C2, C3, and C4 of the biological tissue 60 by using the three-dimensional data 52. The image processing device 11 sets a pair of planes intersecting at a single line L1 passing through the positions of the centroids B1, B2, B3, and B4 as cutting planes P1 and P2. The image processing device 11 forms, in the three-dimensional data 52, an opening exposing the lumen of the biological tissue 60 from a region interposed between the cutting planes P1 and P2 in the three-dimensional image 53.

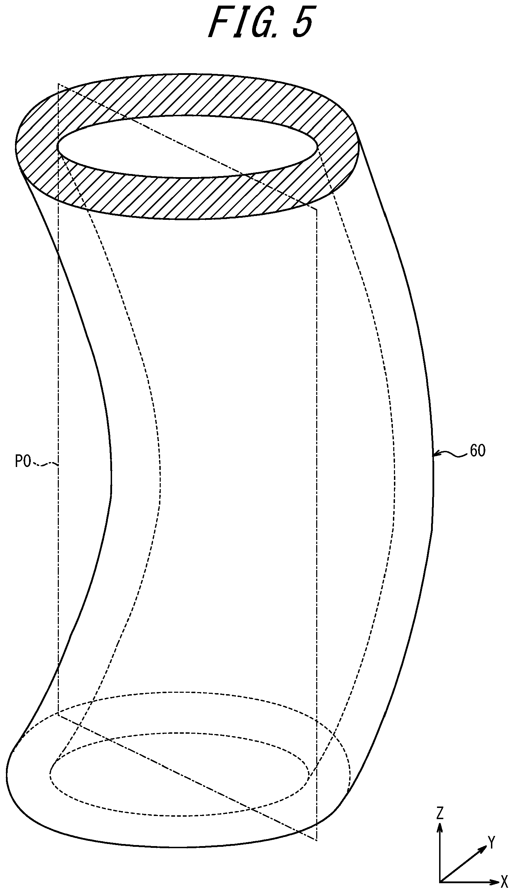

In the case of a three-dimensional model of the bent blood vessel as illustrated in FIG. 4, when the three-dimensional model is cut with one plane to display the lumen, there is a case in which a cutting plane P0 cannot correctly display the inside of the blood vessel as illustrated in FIG. 5. In the present embodiment, as illustrated in FIG. 4, by continuously capturing centroids of the blood vessel, the three-dimensional model can be cut such that the inside of the blood vessel can be reliably displayed.

In FIG. 4, for convenience, four cross sections C1, C2, C3, and C4 are illustrated as the plurality of cross sections in the lateral direction of the lumen of the biological tissue 60, but the number of cross sections serving as calculation targets of the centroid positions is not limited to four, and is preferably the same as the number of cross-sectional images acquired by IVUS.

A configuration of an image processing system 10 according to the present embodiment will be described with reference to FIG. 1.

The image processing system 10 includes the image processing device 11, a cable 12, a drive unit 13, a keyboard 14, a mouse 15, and the display 16.

The image processing device 11 is a dedicated computer specialized for image diagnosis in the present embodiment, but may also be a general-purpose computer such as a PC. The term "PC" is an abbreviation for personal computer.

The cable 12 is used to connect the image processing device 11 and the drive unit 13.

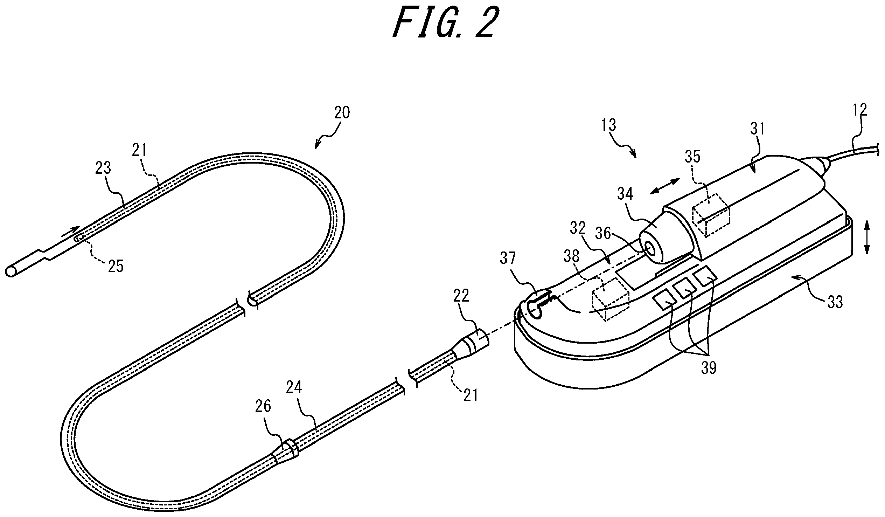

The drive unit 13 is a device to be used by connecting to a probe 20 illustrated in FIG. 2 to drive the probe 20. The drive unit 13 is also referred to as an MDU. The term "MDU" is an abbreviation for motor drive unit. The probe 20 is applied to IVUS. The probe 20 is also referred to as an IVUS catheter or an image diagnostic catheter.

The keyboard 14, the mouse 15, and the display 16 are connected to the image processing device 11 via any cable or wirelessly. The display 16 is, for example, an LCD, an organic EL display, or an HMD. The term "LCD" is an abbreviation for liquid crystal display. The term "EL" is an abbreviation for electro luminescence. The term "HMD" is an abbreviation for head-mounted display.

The image processing system 10 optionally further includes a connection terminal 17 and a cart unit 18.

The connection terminal 17 is used to connect the image processing device 11 and an external device. The connection terminal 17 is, for example, a USB terminal. The term "USB" is an abbreviation for universal serial bus. The external device is, for example, a recording medium such as a magnetic disc drive, a magneto-optical disc drive, or an optical disc drive.

The cart unit 18 is a cart equipped with casters for movement. The image processing device 11, the cable 12, and the drive unit 13 are disposed on a cart body of the cart unit 18. The keyboard 14, the mouse 15, and the display 16 are disposed on an uppermost table of the cart unit 18.

Configurations of the probe 20 and the drive unit 13 according to the present embodiment will be described with reference to FIG. 2.

The probe 20 includes a drive shaft 21, a hub 22, a sheath 23, an outer tube 24, an ultrasound transducer 25, and a relay connector 26.

The drive shaft 21 passes through the sheath 23 to be inserted into a body cavity of a living body and the outer tube 24 connected to a proximal end of the sheath 23, and extends to an inside of the hub 22 provided at a proximal end of the probe 20. The drive shaft 21 is provided with the ultrasound transducer 25, which transmits and receives signals, at a distal end thereof, and is rotatably provided in the sheath 23 and the outer tube 24. The relay connector 26 connects the sheath 23 and the outer tube 24.

The hub 22, the drive shaft 21, and the ultrasound transducer 25 are connected to each other to integrally move forward and backward in an axial direction. Therefore, for example, when the hub 22 is pressed toward a distal side, the drive shaft 21 and the ultrasound transducer 25 move inside the sheath 23 toward the distal side. For example, when the hub 22 is pulled toward a proximal side, the drive shaft 21 and the ultrasound transducer 25 move inside the sheath 23 toward the proximal side as indicated by an arrow.

The drive unit 13 includes a scanner unit 31, a slide unit 32, and a bottom cover 33.

The scanner unit 31 is connected to the image processing device 11 via the cable 12. The scanner unit 31 includes a probe connection section 34 connected to the probe 20, and a scanner motor 35 which is a drive source for rotating the drive shaft 21.

The probe connection section 34 is freely detachably connected to the probe 20 through an insertion port 36 of the hub 22 provided at the proximal end of the probe 20. Inside the hub 22, a proximal end of the drive shaft 21 is rotatably supported, and a rotational force of the scanner motor 35 is transmitted to the drive shaft 21. A signal is transmitted and received between the drive shaft 21 and the image processing device 11 via the cable 12. In the image processing device 11, generation of a tomographic image of a body lumen and image processing are executed based on the signal transmitted from the drive shaft 21.

The slide unit 32 is mounted with the scanner unit 31 in a manner of being capable of moving forward and backward, and is mechanically and electrically connected to the scanner unit 31. The slide unit 32 includes a probe clamp section 37, a slide motor 38, and a switch group 39.

The probe clamp section 37 is disposed coaxially with the probe connection section 34 on the distal side relative to the probe connection section 34, and supports the probe 20 to be connected to the probe connection section 34.

The slide motor 38 is a drive source that generates a driving force in the axial direction. The scanner unit 31 moves forward and backward when driven by the slide motor 38, and the drive shaft 21 moves forward and backward in the axial direction accordingly. The slide motor 38 is, for example, a servo motor.

The switch group 39 includes, for example, a forward switch and a pull-back switch that are pressed when the scanner unit 31 is to be moved forward or backward, and a scan switch that is pressed when image drawing is to be started or ended. Various switches may be included in the switch group 39 as necessary without being limited to the example here.

When the forward switch is pressed, the slide motor 38 rotates forward, and the scanner unit 31 moves forward. Meanwhile, when the pull-back switch is pressed, the slide motor 38 rotates backward, and the scanner unit 31 moves backward.

When the scan switch is pressed, the image drawing is started, the scanner motor 35 is driven, and the slide motor 38 is driven to move the scanner unit 31 backward. The user such as the operator connects the probe 20 to the scanner unit 31 in advance, such that the drive shaft 21 rotates and moves toward the proximal side in the axial direction upon the start of the image drawing. When the scan switch is pressed again, the scanner motor 35 and the slide motor 38 are stopped, and the image drawing is ended.

The bottom cover 33 covers a bottom and an entire circumference of a side surface on a bottom side of the slide unit 32, and is capable of moving toward and away from the bottom of the slide unit 32.

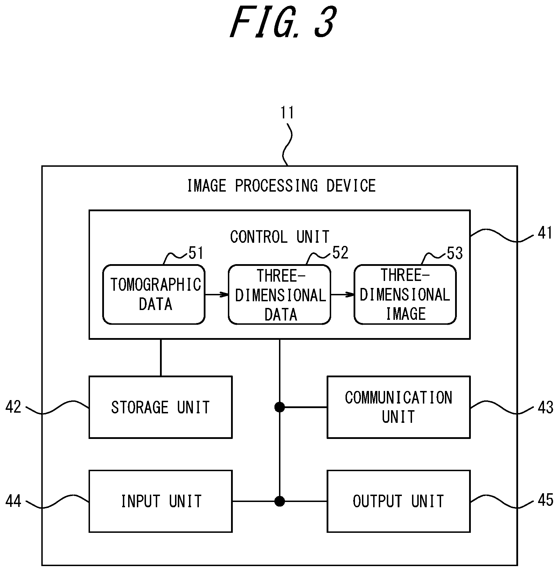

A configuration of the image processing device 11 will be described with reference to FIG. 3.

The image processing device 11 includes a control unit 41, a storage unit 42, a communication unit 43, an input unit 44, and an output unit 45.

The control unit 41 includes at least one processor, at least one dedicated circuit, or a combination thereof. The processor is a general-purpose processor such as a CPU or GPU, or a dedicated processor specialized for specific processing. The term "CPU" is an abbreviation for central processing unit. The term "GPU" is an abbreviation for graphics processing unit. The dedicated circuit is, for example, an FPGA or an ASIC. The term "FPGA" is an abbreviation for field-programmable gate array. The term "ASIC" is an abbreviation for application specific integrated circuit. The control unit 41 executes processing related to an operation of the image processing device 11 while controlling each unit of the image processing system 10 including the image processing device 11.

The storage unit 42 includes at least one semiconductor memory, at least one magnetic memory, at least one optical memory, or a combination of at least two thereof. The semiconductor memory is, for example, a RAM or a ROM. The term "RAM" is an abbreviation for random access memory. The term "ROM" is an abbreviation for read only memory. The RAM is, for example, an SRAM or a DRAM. The term "SRAM" is an abbreviation for static random access memory. The term "DRAM" is an abbreviation for dynamic random access memory. The ROM is, for example, an EEPROM. The term "EEPROM" is an abbreviation for electrically erasable programmable read only memory. The storage unit 42 functions as, for example, a main storage device, an auxiliary storage device, or a cache memory. The storage unit 42 stores data used for the operation of the image processing device 11, such as tomographic data 51, and data obtained by the operation of the image processing device 11, such as the three-dimensional data 52 and the three-dimensional image 53.

The communication unit 43 includes at least one communication interface. The communication interface is, for example, a wired LAN interface, a wireless LAN interface, or an image diagnostic interface for receiving IVUS signals and executing A/D conversion for the IVUS signals. The term "LAN" is an abbreviation for local area network. The term "A/D" is an abbreviation for analog to digital. The communication unit 43 receives the data used for the operation of the image processing device 11 and transmits the data obtained by the operation of the image processing device 11. In the present embodiment, the drive unit 13 is connected to the image diagnostic interface included in the communication unit 43.

The input unit 44 includes at least one input interface. The input interface is, for example, a USB interface, an HDMI (registered trademark) interface, or an interface compatible with short-range wireless communication such as Bluetooth (registered trademark). The term "HDMI" (registered trademark) is an abbreviation for High-Definition Multimedia Interface. The input unit 44 receives an operation by the user such as an operation of inputting data used for the operation of the image processing device 11. In the present embodiment, the keyboard 14 and the mouse 15 are connected to the USB interface or the interface compatible with short-range wireless communication included in the input unit 44. When a touch screen is provided integrally with the display 16, the display 16 may be connected to the USB interface or the HDMI (registered trademark) interface included in the input unit 44.

The output unit 45 includes at least one output interface. The output interface is, for example, a USB interface, an HDMI (registered trademark) interface, or an interface compatible with short-range wireless communication such as Bluetooth (registered trademark). The output unit 45 outputs the data obtained by the operation of the image processing device 11. In the present embodiment, the display 16 is connected to the USB interface or the HDMI (registered trademark) interface included in the output unit 45.

A function of the image processing device 11 is implemented by executing an image processing program according to the present embodiment by the processor corresponding to the control unit 41. That is, the function of the image processing device 11 is implemented by software. The image processing program causes a computer to function as the image processing device 11 by causing the computer to execute processing of the image processing device 11. That is, the computer functions as the image processing device 11 by executing the processing of the image processing device 11 according to the image processing program.

The program may be stored in a non-transitory computer-readable medium in advance. The non-transitory computer-readable medium is, for example, a flash memory, a magnetic recording device, an optical disc, a magneto-optical recording medium, or a ROM. Distribution of the program is executed by, for example, selling, transferring, or lending a portable medium such as an SD card, a DVD or a CD-ROM storing the program. The term "SD" is an abbreviation for secure digital. The term "DVD" is an abbreviation for digital versatile disc. The term "CD-ROM" is an abbreviation for compact disc read only memory. The program may be distributed by storing the program in a storage of a server in advance and transferring the program from the server to another computer. The program may be provided as a program product.

For example, the computer temporarily stores, in the main storage device, the program stored in the portable medium or the program transferred from the server. The computer reads, by the processor, the program stored in the main storage device, and executes, by the processor, processing according to the read program. The computer may read the program directly from the portable medium and execute the processing according to the program. Each time the program is transferred from the server to the computer, the computer may sequentially execute processing according to the received program. The processing may be executed by a so-called ASP type service in which the function is implemented only by execution instruction and result acquisition without transferring the program from the server to the computer. The term "ASP" is an abbreviation for application service provider. The program includes information provided for processing by an electronic computer and conforming to the program. For example, data that is not a direct command to the computer but has a property that defines the processing of the computer corresponds to the "information conforming to the program".

The functions of the image processing device 11 may be partially or entirely implemented by the dedicated circuit corresponding to the control unit 41. That is, the functions of the image processing device 11 may be partially or entirely implemented by hardware.

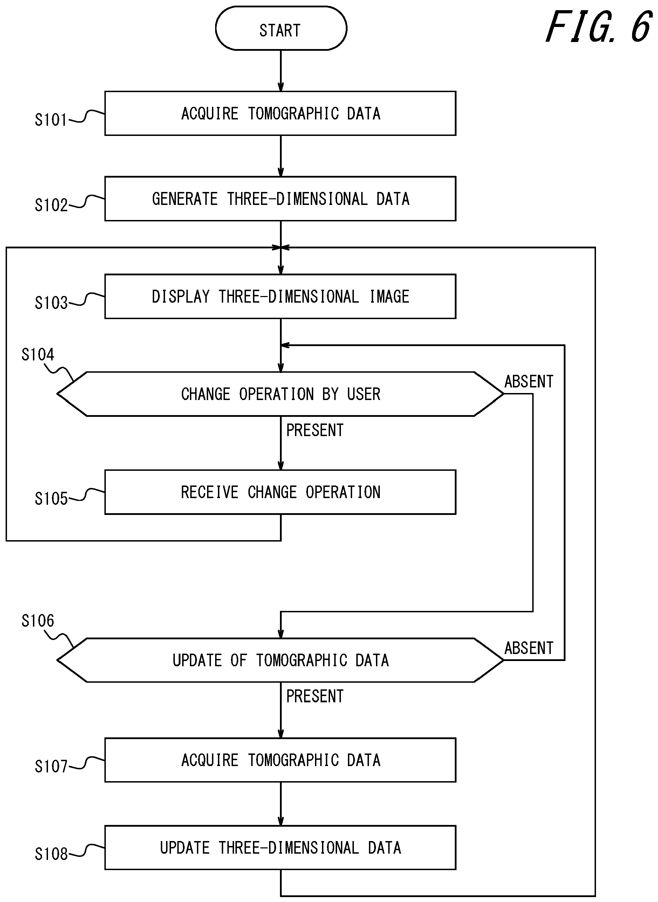

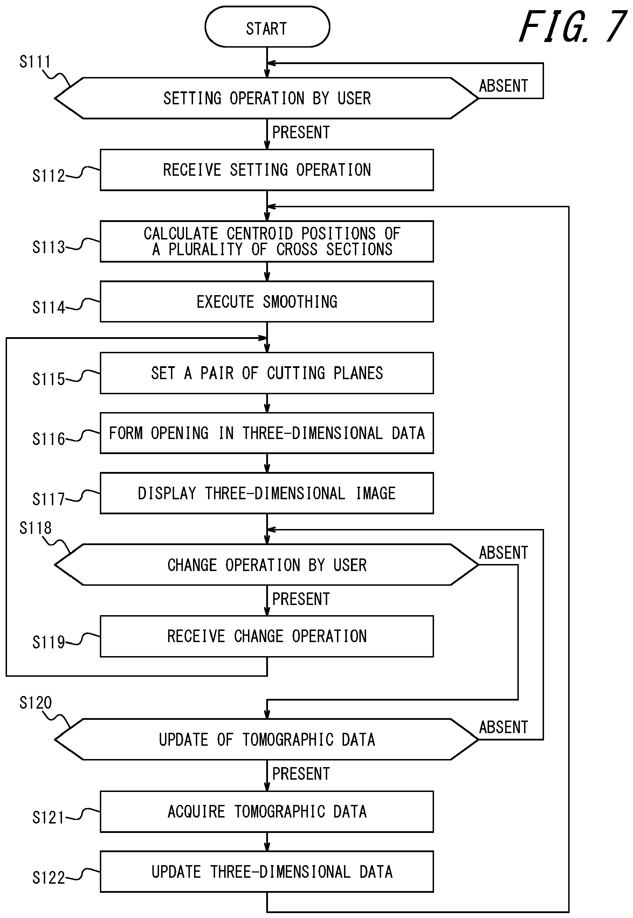

An operation of the image processing system 10 according to the present embodiment will be described with reference to FIGS. 6 and 7. The operation of the image processing system 10 corresponds to an image display method according to the present embodiment.

Before a start of a flow in FIG. 6, the probe 20 is primed by the user. Thereafter, the probe 20 is fitted into the probe connection section 34 and the probe clamp section 37 of the drive unit 13, and is connected and fixed to the drive unit 13. Then, the probe 20 is inserted to a target site in the biological tissue 60 such as the blood vessel or the heart.

In step S101, the scan switch included in the switch group 39 is pressed, and a so-called pull-back operation is executed by pressing the pull-back switch included in the switch group 39. The probe 20 transmits an ultrasound wave inside the biological tissue 60 by the ultrasound transducer 25 that moves backward in the axial direction by the pull-back operation. The ultrasound transducer 25 radially transmits the ultrasound wave while moving inside the biological tissue 60. The ultrasound transducer 25 receives a reflected wave of the transmitted ultrasound wave. The probe 20 inputs a signal of the reflected wave received by the ultrasound transducer 25 to the image processing device 11. The control unit 41 of the image processing device 11 processes the input signal to sequentially generate cross-sectional images of the biological tissue 60, thereby acquiring the tomographic data 51, which includes a plurality of cross-sectional images.

Specifically, the probe 20 transmits the ultrasound wave in a plurality of directions from a rotation center to an outside by the ultrasound transducer 25 while causing the ultrasound transducer 25 to rotate in a circumferential direction and to move in the axial direction inside the biological tissue 60. The probe 20 receives the reflected wave from a reflecting object present in each of the plurality of directions inside the biological tissue 60 by the ultrasound transducer 25. The probe 20 transmits the signal of the received reflected wave to the image processing device 11 via the drive unit 13 and the cable 12. The communication unit 43 of the image processing device 11 receives the signal transmitted from the probe 20. The communication unit 43 executes A/D conversion for the received signal. The communication unit 43 inputs the A/D-converted signal to the control unit 41. The control unit 41 processes the input signal to calculate an intensity value distribution of the reflected wave from the reflecting object present in a transmission direction of the ultrasound wave of the ultrasound transducer 25. The control unit 41 sequentially generates two-dimensional images having a luminance value distribution corresponding to the calculated intensity value distribution as the cross-sectional images of the biological tissue 60, thereby acquiring the tomographic data 51 which is a data set of the cross-sectional images. The control unit 41 stores the acquired tomographic data 51 in the storage unit 42.

In the present embodiment, the signal of the reflected wave received by the ultrasound transducer 25 corresponds to raw data of the tomographic data 51, and the cross-sectional images generated by processing the signal of the reflected wave by the image processing device 11 correspond to processed data of the tomographic data 51.

In a modification of the present embodiment, the control unit 41 of the image processing device 11 may store the signal input from the probe 20 as it is in the storage unit 42 as the tomographic data 51. Alternatively, the control unit 41 may store data indicating the intensity value distribution of the reflected wave calculated by processing the signal input from the probe 20 in the storage unit 42 as the tomographic data 51. That is, the tomographic data 51 is not limited to the data set of the cross-sectional images of the biological tissue 60, and may be data representing a cross section of the biological tissue 60 at each moving position of the ultrasound transducer 25 in any format.

In a modification of the present embodiment, an ultrasound transducer that transmits the ultrasound wave in the plurality of directions without rotating may be used instead of the ultrasound transducer 25 that transmits the ultrasound wave in the plurality of directions while rotating in the circumferential direction.

In a modification of the present embodiment, the tomographic data 51 may be acquired using OFDI or OCT instead of being acquired by using IVUS. The term "OFDI" is an abbreviation for optical frequency domain imaging. The term "OCT" is an abbreviation for optical coherence tomography. When OFDI or OCT is used, as a sensor that acquires the tomographic data 51 while moving in the lumen of the biological tissue 60, a sensor that acquires the tomographic data 51 by emitting light in the lumen of the biological tissue 60 is used instead of the ultrasound transducer 25 that acquires the tomographic data 51 by transmitting the ultrasound wave in the lumen of the biological tissue 60.

In a modification of the present embodiment, instead of the image processing device 11 generating the data set of the cross-sectional images of the biological tissue 60, another device may generate the same data set, and the image processing device 11 may acquire the data set from the other device. That is, instead of the control unit 41 of the image processing device 11 processing the IVUS signal to generate the cross-sectional images of the biological tissue 60, another device may process the IVUS signal to generate the cross-sectional images of the biological tissue 60 and input the generated cross-sectional images to the image processing device 11.

In step S102, the control unit 41 of the image processing device 11 generates the three-dimensional data 52 of the biological tissue 60 based on the tomographic data 51 acquired in step S101. Note that at this time, if already generated three-dimensional data 52 is present, it is preferable to update only data at a location corresponding to the updated tomographic data 51, instead of regenerating all the three-dimensional data 52 from the beginning. Accordingly, a data processing amount when generating the three-dimensional data 52 can be reduced, and a real-time property of the three-dimensional image 53 in the subsequent step S103 can be improved.

Specifically, the control unit 41 of the image processing device 11 generates the three-dimensional data 52 of the biological tissue 60 by stacking the cross-sectional images of the biological tissue 60 included in the tomographic data 51 stored in the storage unit 42, and converting the same into three-dimensional data. As a method for three-dimensional conversion, any method among a rendering method such as surface rendering or volume rendering, and various processing such as texture mapping including environment mapping, and bump mapping, which is associated with the rendering method, is used. The control unit 41 stores the generated three-dimensional data 52 in the storage unit 42.

In step S103, the control unit 41 of the image processing device 11 displays the three-dimensional data 52 generated in step S102 on the display 16 as the three-dimensional image 53. The control unit 41 may set an angle for displaying the three-dimensional image 53 to any angle.

Specifically, the control unit 41 of the image processing device 11 generates the three-dimensional image 53 based on the three-dimensional data 52 stored in the storage unit 42. The control unit 41 displays the generated three-dimensional image 53 on the display 16 via the output unit 45.

In step S104, if there is an operation of setting the angle for displaying the three-dimensional image 53 as a change operation by the user, processing of step S105 is executed. If there is no change operation by the user, processing of step S106 is executed.

In step S105, the control unit 41 of the image processing device 11 receives, via the input unit 44, the operation of setting the angle for displaying the three-dimensional image 53. The control unit 41 adjusts the angle for displaying the three-dimensional image 53 to the set angle. In step S103, the control unit 41 causes the display 16 to display the three-dimensional image 53 at the angle set in step S105.

Specifically, the control unit 41 of the image processing device 11 receives, via the input unit 44, an operation by the user of rotating the three-dimensional image 53 displayed on the display 16 by using the keyboard 14, the mouse 15, or the touch screen provided integrally with the display 16. The control unit 41 interactively adjusts the angle for displaying the three-dimensional image 53 on the display 16 according to the operation by the user. Alternatively, the control unit 41 receives, via the input unit 44, an operation by the user of inputting a numerical value of the angle for displaying the three-dimensional image 53 by using the keyboard 14, the mouse 15, or the touch screen provided integrally with the display 16. The control unit 41 adjusts the angle for displaying the three-dimensional image 53 on the display 16 in accordance with the input numerical value.

In step S106, if the tomographic data 51 is updated, processing of step S107 and step S108 is executed. If the tomographic data 51 is not updated, the presence or absence of the change operation by the user is confirmed again in step S104.

In step S107, similarly to the processing of step S101, the control unit 41 of the image processing device 11 processes the signal input from the probe 20 to newly generate cross-sectional images of the biological tissue 60, thereby acquiring the tomographic data 51 including at least one new cross-sectional image.

In step S108, the control unit 41 of the image processing device 11 updates the three-dimensional data 52 of the biological tissue 60 based on the tomographic data 51 acquired in step S107. Then, in step S103, the control unit 41 displays the three-dimensional data 52 updated in step S108 on the display 16 as the three-dimensional image 53. Note that in step S108, it is preferable to update only data at a location corresponding to the updated tomographic data 51. Accordingly, the data processing amount when generating the three-dimensional data 52 can be reduced, and the real-time property of the three-dimensional image 53 can be improved in step S108.

In step S111, if there is an operation of setting an angle between the cutting planes P1 and P2 as illustrated in FIG. 4 as a setting operation by the user, processing of step S112 is executed.

In step S112, the control unit 41 of the image processing device 11 receives, via the input unit 44, the operation of setting the angle between the cutting planes P1 and P2.

Specifically, the control unit 41 of the image processing device 11 receives, via the input unit 44, an operation by the user of inputting a numerical value of the angle between the cutting planes P1 and P2 by using the keyboard 14, the mouse 15, or the touch screen provided integrally with the display 16.

In step S113, the control unit 41 of the image processing device 11 calculates the centroid positions of the plurality of cross sections in the lateral direction of the lumen of the biological tissue 60 by using the latest three-dimensional data 52 stored in the storage unit 42. The latest three-dimensional data 52 is the three-dimensional data 52 generated in step S102 if the processing of step S108 is not executed, and is the three-dimensional data 52 updated in step S108 if the processing of step S108 is executed. Note that at this time, if the already generated three-dimensional data 52 is present, it is preferable to update only data at a location corresponding to the updated tomographic data 51, instead of regenerating all the three-dimensional data 52 from the beginning. Accordingly, the data processing amount when generating the three-dimensional data 52 can be reduced, and the real-time property of the three-dimensional image 53 in a subsequent step S117 can be improved.

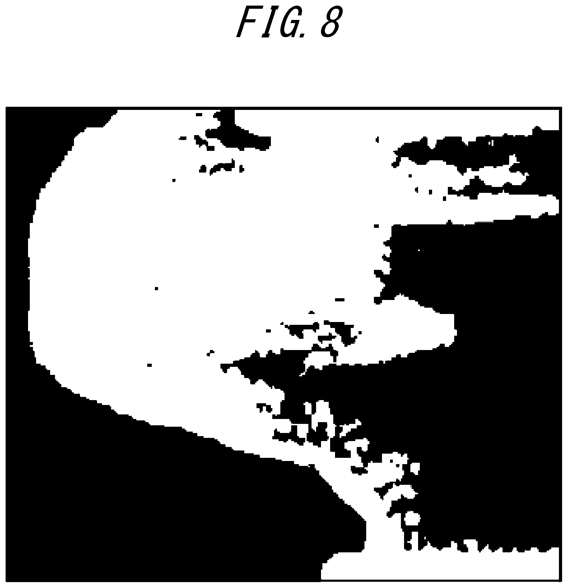

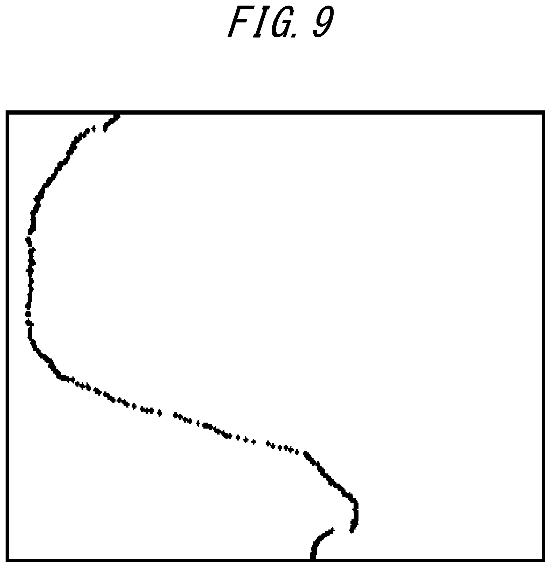

Specifically, as illustrated in FIG. 8, if the control unit 41 of the image processing device 11 generates a corresponding new cross-sectional image in step S107 for each of the plurality of cross-sectional images generated in step S101, the control unit 41 replaces each of the plurality of cross-sectional images generated in step S101 with the new cross-sectional image, and then binarizes the cross-sectional image. As illustrated in FIG. 9, the control unit 41 extracts a point cloud on an inner surface of the biological tissue 60 from the binarized cross-sectional image. For example, the control unit 41 extracts a point cloud on an inner surface of a blood vessel by extracting points corresponding to an inner surface of a main blood vessel one by one along a vertical direction of the cross-sectional image having an r-axis as a horizontal axis and a θ-axis as a vertical axis. The control unit 41 may simply obtain a centroid of the extracted point cloud on the inner surface, but in this case, since the point cloud is not uniformly sampled over the inner surface, a centroid position shifts. Therefore, in the present embodiment, the control unit 41 calculates a convex hull of the extracted point cloud on the inner surface, and calculates a centroid position Cₙ = (Cₓ, C_{y}) by using a formula for obtaining a centroid of a polygon as follows. However, in the following formula, it is assumed that n vertices (x₀, y₀), (x₁, y₁),..., (xₙ₋₁, yₙ₋₁) are present on the convex hull counterclockwise as the point cloud on the inner surface as illustrated in FIG. 9, and (xₙ, yₙ) is regarded as (x₀, y₀).

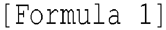

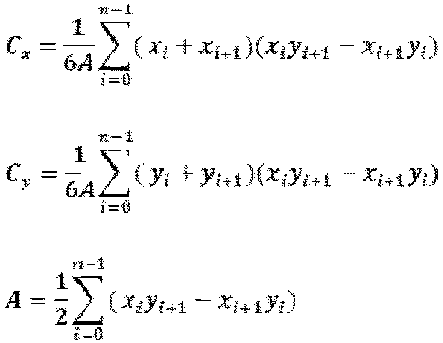

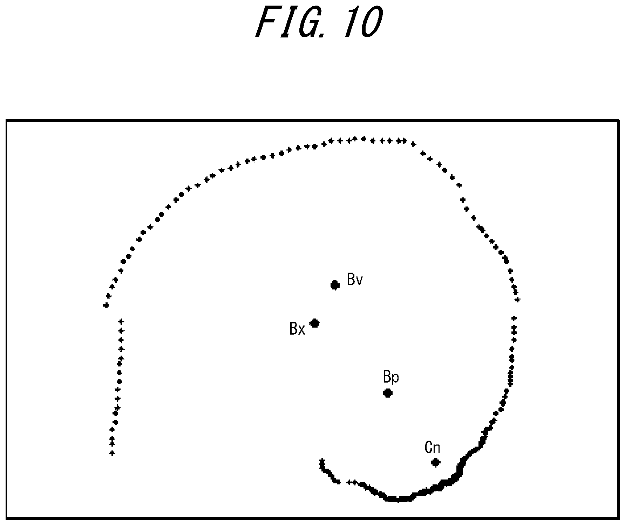

The centroid positions obtained as results are illustrated in FIG. 10. In FIG. 10, a point Cn is a center of the cross-sectional image. A point Bp is a centroid of the point cloud on the inner surface. A point Bv is a centroid of the vertices of the polygon. A point Bx is a centroid of the polygon serving as the convex hull.

As a method for calculating the centroid position of the blood vessel, a method other than the method of calculating the centroid position of the polygon serving as the convex hull may be used. For example, with respect to an original cross-sectional image that is not binarized, a method of calculating a center position of a maximum circle that falls within the main blood vessel as the centroid position may be used. Alternatively, with respect to the binarized cross-sectional image having the r-axis as the horizontal axis and the θ-axis as the vertical axis, a method of calculating an average position of pixels in a main blood vessel region as the centroid position may be used. The same method as described above may also be used when the biological tissue 60 is not a blood vessel.

In step S114, the control unit 41 of the image processing device 11 smooths calculation results of the centroid positions in step S113.

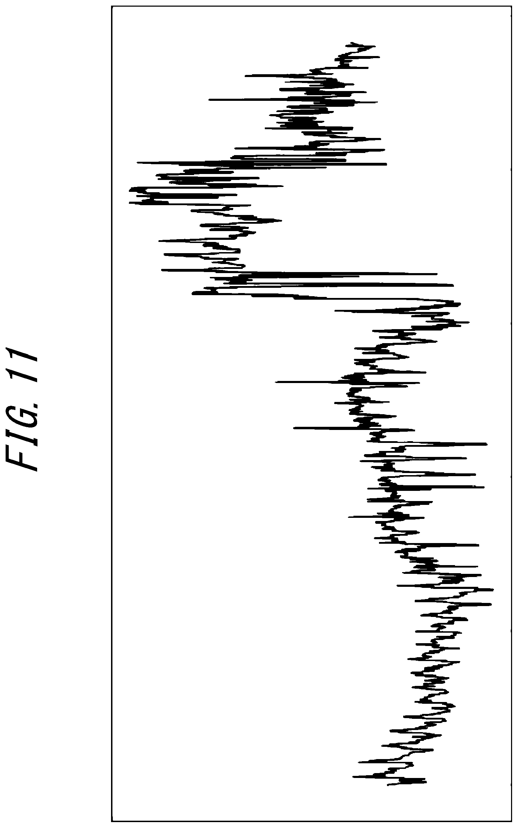

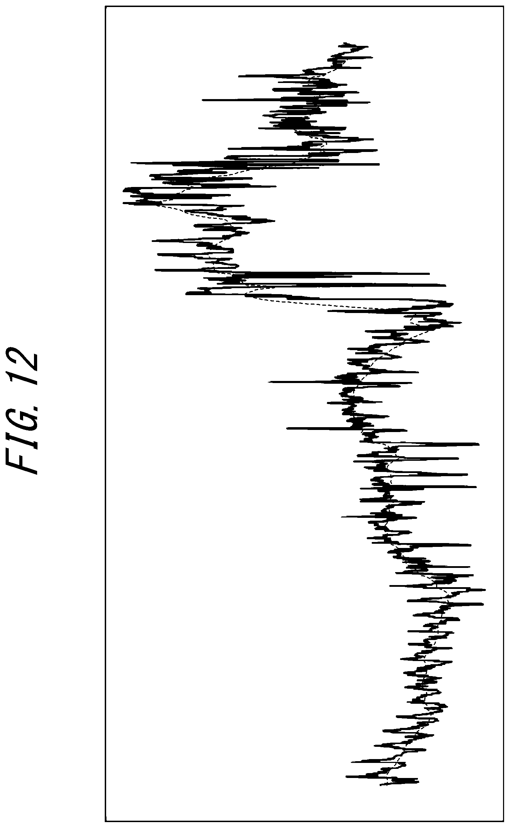

As illustrated in FIG. 11, when the calculation results of the centroid positions are viewed as a time function, it can be seen that an influence of pulsation is large. Therefore, in the present embodiment, the control unit 41 of the image processing device 11 smooths the calculation results of the centroid positions by using moving average as indicated by a broken line in FIG. 12.

As a smoothing method, a method other than moving average may be used. For example, exponential smoothing method, kernel method, local regression, Ramer-Douglas-Peucker algorithm, Savitzky-Golay method, smoothing spline, or SGM may be used. Alternatively, a method of executing a fast Fourier transform and then removing a high frequency component may be used. Alternatively, Kalman filter or a low-pass filter such as Butterworth filter, Chebyshev filter, digital filter, elliptic filter, or KZ filter may be used. The term "SGM" is an abbreviation for stretched grid method. The term "KZ" is an abbreviation for Kolmogorov-Zurbenko.

If smoothing is simply executed, the centroid positions may enter the tissue. In this case, the control unit 41 may divide the calculation results of the centroid positions according to positions of the plurality of lateral cross sections of the lumen of the biological tissue 60 in the longitudinal direction of the lumen of the biological tissue 60, and may smooth each of the divided calculation results. That is, when a curve of the centroid positions as indicated by the broken line in FIG. 12 overlaps a tissue region, the control unit 41 may divide the curve of the centroid positions into a plurality of sections and smooth each section. Alternatively, the control unit 41 may adjust a degree of smoothing to be executed for the calculation results of the centroid positions according to the positions of the plurality of lateral cross sections of the lumen of the biological tissue 60 in the longitudinal direction of the lumen of the biological tissue 60. That is, when the curve of the centroid positions as indicated by the broken line in FIG. 12 overlaps the tissue region, the control unit 41 may decrease the degree of smoothing to be executed for a part of a section including the overlapping points.

In step S115, the control unit 41 of the image processing device 11 sets a pair of planes intersecting at the single line L1 passing through the centroid positions calculated in step S113, as the cutting planes P1 and P2. In the present embodiment, the control unit 41 smooths the calculation results of the centroid positions in step S114 and then sets the cutting planes P1 and P2, but the processing of step S114 may be omitted.

Specifically, the control unit 41 of the image processing device 11 sets a curve of the centroid positions obtained as a result of the smoothing in step S114 as the line L1. The control unit 41 sets a pair of planes intersecting at the set line L1 and forming the angle set in step S112 as the cutting planes P1 and P2. The control unit 41 specifies three-dimensional coordinates intersecting with the cutting planes P1 and P2 of the biological tissue 60 in the latest three-dimensional data 52 stored in the storage unit 42 as three-dimensional coordinates of an edge of the opening exposing the lumen of the biological tissue 60 in the three-dimensional image 53. The control unit 41 stores the specified three-dimensional coordinates in the storage unit 42. Positions of the cutting planes P1 and P2 may be set freely, but in the present embodiment, the positions are set such that the opening is positioned facing forward in a screen of the display 16.

In step S116, the control unit 41 of the image processing device 11 forms, in the three-dimensional data 52, the opening exposing the lumen of the biological tissue 60 from the region interposed between the cutting planes P1 and P2 in the three-dimensional image 53.

Specifically, the control unit 41 of the image processing device 11 hides a portion in the latest three-dimensional data 52 stored in the storage unit 42 that is specified by the three-dimensional coordinates stored in the storage unit 42 or sets the portion to be transparent when the three-dimensional image 53 is to be displayed on the display 16.

In step S117, the control unit 41 of the image processing device 11 displays the three-dimensional data 52 having the opening formed in step S116 on the display 16 as the three-dimensional image 53.

Specifically, the control unit 41 of the image processing device 11 generates the three-dimensional image 53 in which the portion specified by the three-dimensional coordinates stored in the storage unit 42 is hidden or transparent. The control unit 41 displays the generated three-dimensional image 53 on the display 16 via the output unit 45. Accordingly, the user can virtually observe an inner wall surface of the biological tissue 60 by looking into the biological tissue 60 through the opening.

In step S118, as the change operation by the user, if there is an operation of setting the angle between the cutting planes P1 and P2, or an operation of setting the angle for displaying the three-dimensional image 53, processing of step S119 is executed. If there is no change operation by the user, processing of step S120 is executed.

In step S119, similarly to the processing of step S112, the control unit 41 of the image processing device 11 receives, via the input unit 44, the operation of setting the angle between the cutting planes P1 and P2. In this case, the processing of step S115 and the subsequent steps is executed. Alternatively, similarly to the processing of step S105, the control unit 41 receives, via the input unit 44, the operation of setting the angle for displaying the three-dimensional image 53. The control unit 41 adjusts the angle for displaying the three-dimensional image 53 to the set angle. In this case as well, the processing of step S115 and the subsequent steps is executed. In step S115, the control unit 41 adjusts the positions of the cutting planes P1 and P2 according to the angle set for displaying the three-dimensional image 53. That is, the positions of the cutting planes P1 and P2 are readjusted such that the opening is positioned facing forward in the screen of the display 16.

In step S120, if the tomographic data 51 is updated, processing of step S121 and step S122 is executed. If the tomographic data 51 is not updated, the presence or absence of the change operation by the user is confirmed again in step S118.

In step S121, similarly to the processing of step S101 or step S107, the control unit 41 of the image processing device 11 processes the signal input from the probe 20 to newly generate cross-sectional images of the biological tissue 60, thereby acquiring the tomographic data 51 including at least one new cross-sectional image.

In step S122, the control unit 41 of the image processing device 11 updates the three-dimensional data 52 of the biological tissue 60 based on the tomographic data 51 acquired in step S121. Thereafter, the processing of step S113 and the subsequent steps is executed. Note that in step S122, it is preferable to update only data at a location corresponding to the updated tomographic data 51. Accordingly, the data processing amount when generating the three-dimensional data 52 can be reduced, and the real-time property of data processing after step S113 can be improved.

As described above, in the present embodiment, the control unit 41 of the image processing device 11 causes the display 16 to display, as the three-dimensional image 53, the three-dimensional data 52 representing the biological tissue 60 having the longitudinal lumen. The control unit 41 calculates the centroid positions of the plurality of cross sections in the lateral direction of the lumen of the biological tissue 60 by using the three-dimensional data 52. The control unit 41 sets the pair of planes intersecting at the single line passing through the calculated centroid positions as the cutting planes. The control unit 41 forms, in the three-dimensional data 52, the opening exposing the lumen of the biological tissue 60 from the region interposed between the cutting planes in the three-dimensional image 53.

According to the present embodiment, the user can see the inside of the biological tissue 60 with the three-dimensional image 53. For example, when the user is an operator, it is easy to execute treatment for the inside of the biological tissue 60.

In the present embodiment, when at least a part of the lumen of the biological tissue 60 is bent in the longitudinal direction, the control unit 41 of the image processing device 11 forms, in the three-dimensional data 52, an opening exposing the bent portion of the lumen in the three-dimensional image 53 over the entire longitudinal direction as the opening.

According to the present embodiment, the user can see the inside of the biological tissue 60 on the three-dimensional image 53 without being blocked by an outer wall of the biological tissue 60.

In the present embodiment, the control unit 41 of the image processing device 11 smooths the calculation results of the centroid positions and then sets the cutting planes.

According to the present embodiment, the influence of the pulsation on the calculation results of the centroid positions can be reduced.

The present disclosure is not limited to the above-described embodiment. For example, a plurality of blocks described in the block diagram may be integrated, or one block may be divided. Instead of executing a plurality of steps described in the flowchart in time series according to the description, the steps may be executed in parallel or in a different order according to the processing capability of the device that executes each step or as necessary. In addition, modifications can be made without departing from a gist of the present disclosure.

### Reference Sign List

- 10: image processing system
- 11: image processing device
- 12: cable
- 13: drive unit
- 14: keyboard
- 15: mouse
- 16: display
- 17: connection terminal
- 18: cart unit
- 20: probe
- 21: drive shaft
- 22: hub
- 23: sheath
- 24: outer tube
- 25: ultrasound transducer
- 26: relay connector
- 31: scanner unit
- 32: slide unit
- 33: bottom cover
- 34: probe connection section
- 35: scanner motor
- 36: insertion port
- 37: probe clamp section
- 38: slide motor
- 39: switch group
- 41: control unit
- 42: storage unit
- 43: communication unit
- 44: input unit
- 45: output unit
- 51: tomographic data
- 52: three-dimensional data
- 53: three-dimensional image
- 60: biological tissue

## Claims

1. An image processing device configured to cause a display to display, as a three-dimensional image, three-dimensional data representing a biological tissue having a longitudinal lumen, the image processing device comprising:
a control unit configured to calculate centroid positions of a plurality of cross sections in a lateral direction of the lumen of the biological tissue by using the three-dimensional data, set a pair of planes intersecting at a single line passing through the calculated centroid positions as cutting planes, and form, in the three-dimensional data, an opening exposing the lumen of the biological tissue from a region interposed between the cutting planes in the three-dimensional image.

2. The image processing device according to claim 1, wherein
when at least a part of the lumen of the biological tissue is bent in a longitudinal direction, the control unit forms, in the three-dimensional data, an opening exposing the bent portion of the lumen in the three-dimensional image over the entire longitudinal direction as the opening.

3. The image processing device according to claim 1 or 2, wherein
the control unit is configured to set the cutting planes after smoothing calculation results of the centroid positions.

4. The image processing device according to claim 3, wherein
the control unit is configured to divide the calculation results of the centroid positions according to positions of the plurality of cross sections in a longitudinal direction of the lumen of the biological tissue, and smooth each divided calculation result.

5. The image processing device according to claim 3, wherein
the control unit is configured to adjust a degree of the smoothing executed for the calculation results of the centroid positions according to positions of the plurality of cross sections in a longitudinal direction of the lumen of the biological tissue.

6. The image processing device according to any one of claims 1 to 5, further comprising:
an input unit configured to receive an operation of a user, wherein
the control unit is configured to receive, via the input unit, an operation of setting an angle between the cutting planes.

7. The image processing device according to any one of claims 1 to 5, further comprising:
an input unit configured to receive an operation of a user, wherein
the control unit is configured to receive, via the input unit, an operation of setting an angle for displaying the three-dimensional image, and adjust positions of the cutting planes according to the set angle.

8. The image processing device according to any one of claims 1 to 7, wherein
the biological tissue includes a blood vessel.

9. An image processing system, comprising:
a sensor configured to acquire tomographic data of the biological tissue while moving in the lumen of the biological tissue; and
the image processing device according any one of claims 1 to 8 configured to generate the three-dimensional data based on the tomographic data acquired by the sensor.

10. The image processing system according to claim 9, further comprising:
the display.

11. An image display method for causing a display to display, as a three-dimensional image, three-dimensional data representing a biological tissue having a longitudinal lumen, the image display method comprising:
a computer calculating centroid positions of a plurality of cross sections in a lateral direction of the lumen of the biological tissue by using the three-dimensional data;
the computer setting a pair of planes intersecting at a single line passing through the calculated centroid positions as cutting planes; and
the computer forming, in the three-dimensional data, an opening exposing the lumen of the biological tissue from a region interposed between the cutting planes in the three-dimensional image.

12. An image processing program that causes a computer to execute:
processing of causing a display to display, as a three-dimensional image, three-dimensional data representing a biological tissue having a longitudinal lumen;
processing of calculating centroid positions of a plurality of cross sections in a lateral direction of the lumen of the biological tissue by using the three-dimensional data;
processing of setting a pair of planes intersecting at a single line passing through the calculated centroid positions as cutting planes; and
processing of forming, in the three-dimensional data, an opening exposing the lumen of the biological tissue from a region interposed between the cutting planes in the three-dimensional image.
